# EUROPEAN PATENT APPLICATION

(11) **EP 1 932 540 A1**
(43) Date of publication of application: **18.06.2008**
(21) Application number: 06810093.2
(22) Date of filing: 13.09.2006
(51) Int. Cl.: A61K 39/12, A61K 39/39, A61P 11/00, A61P 31/12

(54) **SECRETORY IgA AND IgG ANTIBODIES-INDUCING AGENT**

(30) Priority: 14.09.2005 JP 2005266881
(71) Applicant: Moriyama, Masami, Yokohama-shi, Kanagawa 232-0064 (JP)
(72) Inventor: MORIYAMA, Masami, Yokohama-shi, Kanagawa 2320064 (JP); HASEGAWA, Hideki Plarm House Kamiigusa 202, Tokyo 1670023 (JP); SATA, Tetsutarou, Tokyo 1360076 (JP); KURATA, Takeshi, Tokyo 1850034 (JP); TANAKA, Toshiaki, Zushi-shi, Kanagawa 2490004 (JP)
(74) Representative: Hiebl, Inge Elisabeth
(86) International application number: PCT/JP2006/318136
(87) International publication number: WO 2007/032376

(57) **Abstract**

A nasal vaccine is provided which induces the production of secretory IgA and IgG antibodies specific for flaviviruses, including hantaviruses. Furthermore, a method for providing protection against infection with flaviviruses by induces of the secretory IgA and IgG antibodies is also provided. The nasal vaccine includes an inactivated antigen of a flavivirus and poly(I:C) or a ceramified powder of surf clam shells as an adjuvant. The vaccine effectively induces secretion of IgA antibodies in the nasal mucosa and serum IgG antibody responses. Also provided is a method for inducing flavivirus-specific IgA and IgG antibodies, including administering an inactivated antigen of a flavivirus and poly(I:C) or a ceramified powder of surf clam shells as an adjuvant to the mucosa of a respiratory tract.

## Description

### TECHNICAL FIELD

The present invention relates to a method for inducing the production of flavivirus-specific secretory IgA and IgG antibodies by nasal administration of an inactivated flavivirus vaccine and an adjuvant. The present invention also relates to a method for providing protection against infection with flaviviruses by nasal administration of an antibody inducer that induces the production of secretory IgA and IgG antibodies, as well as the antibody inducer for inducing secretory IgA and IgG antibodies.

### BACKGROUND ART

Infectious diseases caused by flaviviruses have become an important concern in recent years. Of different members of the family *Flaviviridae,* viruses such as West Nile virus, dengue virus, Japanese encephalitis virus, yellow fever virus, tick-borne encephalitis virus and Hepatitis C virus cause infectious diseases that pose serious problems worldwide.
In addition, infectious diseases caused by hantavirus, a flavivirus, have also become a serious concern. In Japan, the hantavirus infection first came to public attention during the mid-1970's when a number of rat handlers working in local laboratory animal facilities were reported to have fever of unknown origin. The then-unknown disease continued to emerge until 1984: A total of 127 cases had been reported with one death.

An outbreak of the disease occurred before the Second World War in the basin of the Amur River, which forms the border between China and the former Soviet Union. Also, Japan's now-defunct army encountered unknown fever during their occupation of northeastern China (then Manchuria) and reported the disease as epidemic hemorrhagic fever. Later, as many as 3,200 cases of unknown fever were reported among the UN soldiers during the Korean War and attracted significant attention. The causative virus was first isolated by Dr. Lee and colleagues of Korea University, Korea, from Apodemus mice caught in the endemic region. The virus was named as Hantaan virus after the Hantaan River flowing through the region where the mice were caught.

As it turned out, all of these epidemic events were caused by the same agent; hantaan virus belonging to the genus flavivirus. In the 1982 conference held in Japan regarding this disease, the World Health Organization (WHO) collectively named the newly isolated group of similar viruses as hantaan virus, or the genus hantavirus, the new fifth genus of the family *Bunyaviridae.* The WHO also decided to name the disease Hemorrhagic Fever with Renal Syndrome (HFRS).

The hantavirus infection is a zoonotic infection caused by hantavirus that utilizes rodents as natural hosts. Since the virus is excreted in feces and urine of rodents, most infection cases are caused by inhaling the virus in fresh or dried feces or urine. However, some cases have been reported among people who were bitten by rodents. The disease is also considered to be transmitted through nasal, eye or oral contact with infectious materials that have come in contact with rodents.

Hantavirus, the causative agent of HFRS, is prevalent throughout the Eurasian continent and includes as a major species hantaan virus whose primary hosts are Apodemus agrarius, rodents found in Korean peninsula, Northern and Central China and Far East Russia. Each year, tens of thousands of patients are reported in China, several thousands in Russia and several hundreds in Korea. Rattus norvegicus, commonly known as brown rats, are found worldwide and are carriers of Seoul virus. Although no epidemic events have been reported in Japan since the 1984 outbreak that took place in medical laboratory animal facilities, the rats inhabiting harbors throughout the country still remain potential carriers of the virus. Thus, future emergence of patients must be carefully watched.

In 1993, several deaths caused by acute respiratory distress syndrome accompanied by pulmonary edema were reported among Navajo Indians in the South Western United States. Unlike HFRS, the disease was not associated with renal symptoms, but instead led to acute respiratory symptoms, resulting in a mortality of about 50%. Nevertheless, the causative virus of the disease was determined to be hantavirus. The disease, named Hantavirus Pulmonary Syndrome (HPS), was reported not only in North America, but also in South America in 1995.

The hantavirus infections such as HFRS and HPS reported in the North and South America are not considered to be transmitted from human to human. In September 1996, however, 18 cases of HPS were reported in Southern Argentine among local residents and visitors, along with 2 other cases who had contacted the patients but never visited the region. The mortality reached 50%. The fact that none of the patients had a chance to be exposed to rodents suggested human-to-human transmission of the disease, as did virological evidences. The event aroused serious concern, but the epidemic soon ceased and has not reemerged since.

Nonetheless, the threat of airborne hantavirus infections that are transmitted through rat feces, such as HFRS and HPS, still exists: A new outbreak may occur at any time when brown rats and other rodents are prevailing in our living environment throughout the world. In fact, new cases are reported every year in China, Far East Russia and Korea, as mentioned above. Thus, there is a great need for the development of vaccines against hantavirus infection.
The provision of such vaccines against the infectious disease is a particularly urgent matter in China, Far East Russia and Korea where a number of cases are reported each year.

It is known that specific IgA antibodies secreted from the mucosa are highly effective in protecting against influenza and other airborne respiratory infections. IgA antibodies secreted from the mucosa are primarily responsible for the cross-protection against different types of influenza virus. It is believed that IgA antibodies are induced in humans who have been infected with an influenza virus and recovered from it, providing protection against infection with variant viruses of the same subtype.

One way to provide an uninfected individual with protection against infection with viruses and other pathogens is to inoculate the individual with a vaccine, an inactivated antigen of viruses or other pathogens, to deliberately induce an antibody. Examples of such vaccines include influenza vaccine, cholera vaccine, typhoid vaccine, smallpox vaccine and BCG vaccine.

In particular, respiratory diseases such as influenza and severe acute respiratory syndrome (SARS) are caused by infection of respiratory tracts and are thought to be effectively prevented by inducing mucosal immune responses in the respiratory tract by administration of vaccines. Since current vaccines delivered by subcutaneous injection are not capable of inducing mucosal immune responses, there is a need for the development of new, more effective vaccines that can provide cross-protection. One approach to induce secretory IgA antibodies in the respiratory mucosa is nasal administration (inoculation) of antigens. However, the inoculation of antigen alone cannot elicit sufficient immune responses: An adjuvant to augment immune responses needs to be administered together.

In view of the foregoing, like influenza, flavivirus infections, including hantavirus infections such as HFRS and HPS, can be essentially considered respiratory infections.
Thus, successful induction of secretory IgA antibodies in the respiratory mucosa and associated serum IgG antibody responses should provide an effective measure for preventing hantavirus infections and other flavivirus infections.

Although secretory IgA antibodies can be induced in the respiratory mucosa by administering inactivated viral antigens to the mucosa, in particular, the nasal mucosa, the inactivated viral antigens require coadministration of an adjuvant in order to augment their activity as vaccines.
Many adjuvants for such vaccines have been proposed thus far. One example of recently reported adjuvants is poly(I:C), a polynucleotide oligomer of inosinic acid and cytidylic acid (Non-Patent Document 1).
Ceramified powders of surf clam shells have also been reported to act as a natural adjuvant (Patent Document 1).
Patent Document 1: Japanese Patent Application No. 2004-133268
Non-Patent Document 1: J. Clinical Investigation, 110(8), 1175-1184 (2002)

The present inventors have demonstrated that inactivated antigens of flaviviruses, including hantaviruses, nasally administered with the aforementioned specific adjuvants effectively induce secretion of IgA antibodies in the mucosa and serum IgG antibody responses, thus providing protection against infection with fatal doses of flaviviruses, including hantaviruses. This study ultimately led to the present invention.

### DISCLOSURE OF THE INVENTION

### PROBLEMS TO BE SOLVED BY THE INVENTION

Accordingly, it is an object of the present invention to provide a nasal vaccine that uses poly (I:C) and a ceramified powder of surf clam shells as an adjuvant and induces the production of secretory IgA and IgG antibodies specific for flaviviruses, including hantaviruses. It is another object of the present invention as another aspect to provide a method for providing protection against flaviviruses by inducing secretory IgA and IgG antibodies.

### MEANS FOR SOLVING THE PROBLEMS

To achieve the foregoing objects, the present invention in one aspect provides a nasal vaccine that comprises an inactivated antigen of a flavivirus and poly(I:C) or a ceramified powder of surf clam shells as an adjuvant and effectively induces secretion of IgA antibodies in the nasal mucosa and production of serum IgG antibodies.

The present invention in another aspect provides a method for inducing flavivirus-specific IgA and IgG antibodies, comprising administering an inactivated antigen of a flavivirus, along with poly(I:C) or a ceramified powder of surf clam shells as an adjuvant. Specifically, the method for inducing the secretory IgA and IgG antibodies includes administering the inactivated antigen of a flavivirus and the poly(I:C) or a ceramified powder of surf clam shells to the mucosa of the respiratory tract.

### EFFECTS OF THE INVENTION

The nasal vaccine of the present invention, which comprises an inactivated antigen of a flavivirus, including hantaviruses, and poly(I:C) or a ceramified powder of surf clam shells, induces the production of flavivirus-specific secretory IgA antibodies in the mucosa of the respiratory tract as well as flavivirus-specific IgG antibodies.
Secretory IgA antibodies and IgG antibodies are major exocrine pathogen-specific immunoglobulins that play a significant role in protecting the surface of the mucosa from infection with pathogens. These antibodies are abundant in saliva, nasal discharge, secretions from gastrointestinal and respiratory tracts and colostrum. They are also present in serum. The administration of the vaccine of the present invention effectively induces the production of these IgA and IgG antibodies, thus providing protection against infection with flaviviruses.

### BEST MODE FOR CARRYING OUT THE INVENTION

Poly(I:C) used as a vaccine adjuvant in the secretory IgA or IgG antibody inducer provided by the present invention is a double-stranded RNA that serves as a ligand for toll-like receptors (TLRs) and stimulates the innate immune system to provide protective immunity against bacteria, viruses and other pathogenic microorganisms.
When double-stranded RNAs that serve as ligands for TLRs, such as poly(I:C), are administered with a vaccine as an adjuvant, they enhance protection against infection with pathogens not by enhancing the effects of the vaccine, but rather by enhancing the protective immunity against bacteria, viruses and other pathogens, in particular by inducing IgA antibodies and IgG antibodies specific for the viruses and bacteria.

While poly(I:C), the double-stranded RNA for use in the present invention, may differ in molecular size as measured in the number of base pairs (bp), it has been shown that poly(I:C) must have a molecular size of 300 bp or greater in order to effectively stimulate immune responses. Poly(I:C) that is 100 to 1000 bp in size is available from Toray Industries Inc.

The ceramified powder of surf clam shells, one of the vaccine adjuvants used in the secretory IgA and IgG antibody inducer provided by the present invention, is a porous fine powder obtained by calcining surf clam shells. Solutions of this material are known to have sterilizing or bactericidal activity, as well as an ability to remove toxic substances present in trace amounts, such as residual pesticides. Such ceramified surf clam shell powders are available as commercial products.

When observed by electron microscopy, the ceramified surf clam shell powder used as an adjuvant in the present invention appears to be a porous fine powder that is composed of 10 to 100µm non-uniform structures. The porous material acts as a carrier of the vaccine that stimulates antigen-presenting cells.

The vaccine for use in the secretory IgA and IgG antibody inducer provided by the present invention that is administered with the poly(I:C) or the ceramified powder of surf clam shells as an adjuvant is an antigenic suspension or solution generally containing an infectious agent or a fragment or moiety thereof and inducing active immunity when inoculated into the body. The antigenic fragment or moiety that constitutes the vaccine may be a microorganism or a natural product purified from a microorganism (such as viruses and bacteria), or a synthetic or genetically modified protein, peptide, polysaccharide or other products.

One specific example of the live or inactivated vaccine for use in the present invention is live vaccines for flavivirus infections.
As used herein, the term "inactivated antigen" refers to an antigen that has lost its ability to infect new hosts. The term includes virions (complete viral particles), incomplete viral particles, virion components and post-translational modification products thereof, non-virion proteins and post-translational modification products thereof, protective antigens, and neutralized epitopes. These antigens may be inactivated physically (for example, by X-ray irradiation, heat or sonication) or chemically (for example, by formalin, mercury, alcohol or chlorine treatment).

The secretory IgA and IgG antibody inducer provided by the present invention is preferably administered to the mucosa. The mucosa in vertebrates lines the lumen of hollow organs that communicate with the external environment, such as digestive organs, respiratory organs, excretory organs and reproductive organs. The mucosal administration, the preferred administration route of the present invention, therefore includes administration to nasal cavity, oral cavity, vagina, upper respiratory tract and pulmonary alveoli. Administration to the nasal mucosa is particularly preferred. Since the nasal cavity serves as a gateway to respiratory infection by flaviviruses, including hantaviruses, the mucosal administration of the antibody inducer of the present invention provides effective protection against respiratory infections by inducing the epithelial cells of the mucosa to secrete IgA antibodies and inducing the production of serum IgG antibodies.

The pathogens against which the production of secretory IgA and IgG antibodies is induced according to the present invention to provide immune protection are microorganisms that cause diseases or disorders in the hosts. Specifically, these pathogens are flaviviruses including West Nile virus, hantavirus, dengue virus, Japanese encephalitis virus, yellow fever virus, tick-borne encephalitis virus and Hepatitis C virus.

While the dose of the secretory IgA and IgG antibody inducer provided by the present invention may vary depending on the age and weight of the subject and the administration route, the daily dose for an adult is typically in the range of about 10 to 500 mg for oral administration. The daily dose for an adult for mucosal administration, in particular nasal administration, is typically in the range of about 0.1 to 10 mg and preferably in the range of about 0.1 to 1 mg.

### EXAMPLES

The present invention will now be described with reference to specific examples.

### Example 1: Stimulation of IgA and IgG production by synthetic double-stranded RNA poly(I:C)

The ability of an inactivated antigen (an inactivated virus or a subunit antigen) to induce neutralizing antibodies and, thus, induce protection against pathogens was determined in the presence of synthetic double-stranded RNA poly(I:C) as an adjuvant.

### (Materials)

Mice: BALB/c strain (6 week, female)
Virus: Hantavirus strain (obtained from Infectious Disease Surveillance Center (Tokyo))
Vaccine: Hantavirus strain (Infectious Disease Surveillance Center); ether-inactivated vaccine
Adjuvants: CTB* as positive control (CTB (Cholera toxin subunit B) containing 0.1% CT (Cholera toxin)) and poly(I:C)

### (Method)

6-week-old BALB/c mice (Japan SLC, Tokyo) were divided into groups of 5 animals. Each group was inoculated in the nasal cavity with a 5µL solution containing 1 µg of hantavirus vaccine along with 0.1 µg, 1 µg, 3 µg or 10 µg of poly(I:C) adjuvant. After 3 weeks, each group was inoculated in the nasal cavity with the same amount of the vaccine, with or without the adjuvant. After additional 2 weeks, the animals were infected with hantavirus by inoculating a 1.2µL solution containing 100pfu hantavirus in each nasal cavity.
Groups receiving 10 µg or 1 µg of poly(I:C) alone, vaccine alone, or no treatment served as controls.
3 days after the infection, nasal wash and serum were collected. IgA in the nasal wash and IgG in the serum were assayed by ELISA. The virus titer of the nasal wash was determined by a plaque assay using MDCK cells.

Meanwhile, mice were nasally immunized in the same fashion and inoculated with a 20µL solution containing a lethal dose of hantavirus (40LD₅₀, 10^{4.7}EIO⁵⁰ (Approx. 10000 times the amount of virus that can infect 50% embryonated chicken eggs)). The survival rate of the animals was monitored.

To examine the safety of poly(I:C) in the central nervous system, 0.25 µg, 2.5 µg and 25 µg of poly(I:C) in 25µL PBS were inoculated into the brain using a two-step needle. The weight and survival rate of the animals following the inoculation were monitored.
Groups inoculated in the brain with 2.5 µg, 10 µg and 25 µg of CTB* (CTB containing 0.1% CT) in 25µL PBS served as controls.

### (Results)

### (1) Antibody induction and protection against infection by inoculation of nasal hantavirus vaccine with poly(I:C) adjuvant

The mucosal adjuvant activity of poly(I:C) was evaluated. 1µg vaccine and 0.1µg to 10µg poly(I:C) were nasally inoculated 6 weeks previous. The same dose of vaccine, with or without the adjuvant, was nasally inoculated 2 weeks previous. The IgA antibody responses in the nasal mucosa and serum IgG responses were assayed and summarized in Table 1.

**(Table 1)**

| Vaccine | | | | IgA antibody response (nasal wash) Mean (ng/mL) | IgG production (serum) Mean (µg/ML) |
|---|---|---|---|---|---|
| Primary immunization | | Secondary immunization | | | |
| Vaccine (µg) | Poly(I:C) (µg) | Vaccine (µg) | Poly(I:C) (µg) | | |
| 1 | 10 | 1 | 10 | 255.6 | 4.36 |
| 1 | 10 | 1 | - | 92.5 | 2.79 |
| 1 | 3 | 1 | 3 | 62.3 | 1.58 |
| 1 | 3 | 1 | - | 121.8 | 2.56 |
| 1 | 1 | 1 | 1 | 121.9 | 1.58 |
| 1 | 1 | 1 | - | 0.5 | 0.41 |
| 1 | 0.1 | 1 | 0.1 | 14.4 | 1.00 |
| 1 | 0.1 | 1 | - | 9.6 | 0.38 |
| 1 | - | 1 | - | 0 | 0.60 |
| 1 | Denatured 1 | 1 | Denatured 1 | 20.4 | 0.69 |
| - | - | - | - | 0 | 0 |

To determine if the adjuvant activity of poly(I:C) increases in a dose-dependent manner, the dose of poly(I:C) was increased stepwise from 0.1 µg to 10 µg.
As can be seen from the results of Table 1, the IgA antibody responses were induced in the nasal mucosa when 0.1µg or more poly(I:C) was inoculated at the initial immunization.

The results indicate that the amount of IgA induced in the nasal mucosa was dependent on the dose of poly(I:C): The adjuvant activity increased as the dose of poly(I:C) was increased.
When poly(I:C) was used in each of the two immunizations, 100ng/mL or more IgA was secreted in the nasal wash with 1µg dose of poly(I:C). When poly(I:C) was used only in the first immunization, 100ng/mL or more specific IgA antibodies were induced with 3µg dose of poly(I:C).
The serum IgG production was also examined and was shown to be correlated with the IgA secretion: Two immunizations with 1µg vaccine and poly(I:C) delivered at the 4-week interval resulted in the production of 1.6µg/mL serum IgG.

When the animals were infected with hantavirus 2 weeks, after the second immunization with the same immunization conditions, by inoculating a 1.2µL solution containing 100pfu hantavirus in each nasal cavity, the nasal wash of the non-vaccinated control group had a titer of 10² pfu/mL or higher.
In contrast, the viral growth was completely suppressed in the groups receiving two nasal vaccinations each accompanied by poly(I:C). The viral growth was not suppressed both in the group receiving two vaccinations each with 1 µg or more of the vaccine alone and in the group receiving two vaccinations with only the first one given with 3 µg or more of poly(I:C).

The viral growth was significantly suppressed to 10^{0.8} pfu/mL and 10^{1.6} pfu/mL in the groups in which only the first vaccination was accompanied by 1 µg and 0.1 µg of poly(I:C), respectively. The viral growth was not suppressed in the group receiving two vaccinations each comprising the vaccine alone.
These results are summarized in Table 2.

**(Table 2)**

| Vaccine | | | | Virus titer in nasal wash (Mean) (PFU/mL:10ⁿ) |
|---|---|---|---|---|
| Primary immunization | | Secondary immunization | | |
| Vaccine (µg) | Poly(I:C) (µg) | Vaccine (µg) | Poly(I:C) (µg) | |
| 1 | 10 | 1 | 10 | <1* |
| 1 | 10 | 1 | - | <1* |
| 1 | 3 | 1 | 3 | <1* |
| 1 | 3 | 1 | - | <1* |
| 1 | 1 | 1 | 1 | <1* |
| 1 | 1 | 1 | - | 0.8* |
| 1 | 0.1 | 1 | 0.1 | 1.2* |
| 1 | 0.1 | 1 | - | 1.6* |
| 1 | - | 1 | - | 3.0 |
| 1 | Denatured 1 | 1 | Denatured 1 | 2.9 |
| - | - | - | - | 3.1 |

| | | | | |
|---|---|---|---|---|
| *: p < 0.001 | | | | |

### (2) Protection against pneumonia caused by lethal dose of hantavirus by inoculation of nasal vaccine with poly(I:C)

1µg vaccine was nasally inoculated with 10µg, 3µg or 1µg poly(I:C) 6 weeks previous. A booster with vaccine alone was given 2 weeks previous. The animals were then challenged with a 40LD₅₀ dose of hantavirus in a 20µL solution and were examined for the protection against pneumonia.
All the animals died (5/5) within one week in the non-vaccinated group. In this group, the virus titer in the lung reached 10⁶ pfu or higher 3 days after the challenge.
In contrast, all animals survived in each of the vaccinated groups receiving the vaccine along with 1 µg or more of poly(I:C). These results are shown in Table 3.

**(Table 3)**

| Vaccine | | | | Challenge (40LD₅₀) | Lung virus titer (pfu/mL:10ⁿ) | Survived /Total |
|---|---|---|---|---|---|---|
| Primary immunization | | Secondary immunization | | | | |
| Vaccine (µg) | Poly(I:C) (µg) | Vaccine (µg) | Poly(I:C) (µg) | | | |
| 1 | 10 | 1 | 10 | Virus | <0 | 5/5 |
| 1 | 10 | 1 | - | Virus | N.D. | 5/5 |
| 1 | 3 | 1 | - | Virus | N.D. | 5/5 |
| 1 | 1 | 1 | - | Virus | N.D. | 5/5 |
| 1 | CTB* | 1 | CTB* | Virus | <0 | 5/5 |
| - | - | - | - | Virus | 6.1±5.3 | 0/5 |

The foregoing observations demonstrate that poly(I:C) induces mucosal IgA antibody responses by inducing the production of secretory IgA antibodies sufficient for protection against infection.

### Example 2: Protection provided by nasal hantavirus vaccine

### consisting of inactivated viral particles and poly(I:C)

### (Materials)

Vaccine: Ether-treated hantavirus HA vaccine; formalin-inactivated whole particle NC vaccine
Mice: BALB/c strain (6 week, female)

### (Method)

A nasal hantavirus vaccine comprising 0.1 µg of formalin-inactivated whole particle NC hantavirus vaccine and 0.1 µg of poly(I:C) (100 to 1000 bp; Toray Industries Inc.) was administered to BALB/c mice (6 week, female). After 3 weeks, the same vaccine was administered again.
After another week, antibody responses in the nasal wash and serum of mice were determined as indices of mucosal and systemic protective immunity, respectively.

### (Results)

The results are shown in Table 4.

**(Table 4)**

| Primary vaccination (Week 0) | | Secondary vaccination (Week 3) | | Anti-HA NW-IgA (Mean) (2ⁿ) | Anti-HA serum-IgA (Mean) (4ⁿ) |
|---|---|---|---|---|---|
| NC vaccine (0.1µg) | (0.1µg) | NC vaccine (0.1µg) | (0.1µg) | | |
| Ether-treated HA | - | Ether-treated HA | - | 1.4 | 3.1 |
| Ether-treated HA | Poly(I:C) | Ether-treated HA | Poly(I:C) | 1.9 | 4.2 |
| Whole virus particle | - | Whole virus particle | - | 5.0 | 5.3 |
| Whole virus particle | Poly(I:C) | Whole virus particle | Poly(I:C) | 7.8 | 6.2 |
| Ether-treated HA | CTB* | Ether-treated HA | CTB* | 7.1 | 6.9 |

| Primary vaccination (Week 0) | | Secondary vaccination (Week 3) | | Anti-NA NW-IgA (Mean) (2ⁿ) | Anti-NA serum-IgA (Mean) (4ⁿ) |
|---|---|---|---|---|---|
| NC vaccine (0.1µg) | (0.1µg) | NC vaccine (0.1µg) | (0.1µg) | | |
| Ether-treated HA | - | Ether-treated HA | - | 1.1 | 0.9 |
| Ether-treated HA | Poly(I:C) | Ether-treated HA | Poly(I:C) | 1.5 | 2.9 |
| Whole virus particle | - | Whole virus particle | - | 3.5 | 3.7 |
| Whole virus particle | Poly(I:C) | Whole virus particle | Poly(I:C) | 5.4 | 5.4 |
| Ether-treated HA | CTB* | Ether-treated HA | - | 6.2 | 6.2 |

The results of the table indicate that the mucosal and systemic protective immunity was also enhanced by the hantavirus vaccine using inactivated whole viral particles in conjunction with poly(I:C).
It was also demonstrated that 0.1µg vaccine used in conjunction with 0.1µg poly(I:C) elicited responses comparable to those in the positive control group receiving a split-product vaccine and CTB*, a combination that achieves high adjuvant activity to provide effective protection against viral infection.
These responses are even higher than the responses observed with the split-product vaccine combined with poly(I:C).
These observations indicate that the nasal vaccine comprising split-product vaccine or other types of vaccines are similarly effective if used with poly(I:C).

### Example 3: Molecular size of poly(I:C)

### (Materials)

Virus: Hantavirus
Poly(I:C):
Size (L): 1 to 300 bp (Fluka)
Size (M): 100 to 1000 bp (Toray)
Size (H): > 3.3 x 10⁶ bp (Fluka)

Poly(A:U)
Mice: BALB/c strain (6 week, female)

### (Method)

A hantavirus split-product vaccine (0.4 µg) was nasally administered to BALB/c mice (6 week, female) with 0.1µg poly(I:C) in varying sizes. After 3 weeks, the same vaccine was administered again.
After another week, antibody responses against HA and NA in the nasal wash and serum of mice were determined as indices of mucosal and systemic protective immunity, respectively.

### (Results)

The results are shown in Table 5.

**(Table 5)**

| Primary vaccination (Week 0) | | Secondary vaccination (Week 3) | | Anti-HA NW-IgA (Mean) (2ⁿ) | Anti-HA serum-IgA (Mean) (4ⁿ) |
|---|---|---|---|---|---|
| NC vaccine Poly(I:C) (µg) | | NC vaccine Poly(I:C) (µg) | | | |
| 0.4 0.1(M) | | 0.4 0.1(M) | | 3.2 | 5.4 |
| 0.4 0.1(H) | | 0.4 0.1(H) | | 3.3 | 5.8 |
| 0.4 0.1(L) | | 0.4 0.1(L) | | 2.3 | 5.7 |

| Primary vaccination (Week 0) | | Secondary vaccination (Week 3) | | Anti-NA NW-IgA (Mean) (2ⁿ) | Anti-NA serum-IgA (Mean) (4ⁿ) |
|---|---|---|---|---|---|
| NC vaccine (µg) | Poly(I:C) (µg) | NC vaccine (µg) | Poly(I:C) (µg) | | |
| 0.4 | 0.1(M) | 0.4 | 0.1(M) | 2.2 | 4.2 |
| 0.4 | 0.1(H) | 0.4 | 0.1(H) | 2.4 | 4.1 |
| 0.4 | 0.1(L) | 0.4 | 0.1(L) | 1.2 | 3.8 |

As can be seen from the results shown in the table, the groups receiving poly(I:C) with a molecular size of 10 to 300 bp showed lower mucosal responses than the other groups, indicating that poly(I:C) needs to have a molecular size of approximately 300 bp or greater in order to act as an effective adjuvant.

### Example 4: Induction of antibodies and protection against infection by nasal vaccine using ceramified surf clam shell powder as adjuvant

### 1. Materials

The following materials were used:
Mice: BALB/c strain (6 week, female)
Virus: Hantavirus strain (obtained from Infectious Disease Surveillance Center (Tokyo))
Vaccine: Hantavirus strain (Infectious Disease Surveillance Center); ether-inactivated vaccine
Adjuvants: CTB* as positive control (CTB (Cholera toxin subunit B) containing 0.1% CT (Cholera toxin)) and ceramified surf clam shell powder

### 2. Method

6-week-old BALB/c mice were divided into groups of 5 animals. 3µg vaccine in a 5µL solution was nasally inoculated to each animal with 10 µg or 100 µg of the ceramified surf clam shell powder adjuvant. After 3 and 5 weeks, the same doses of the vaccine and the adjuvant were nasally inoculated. After additional 2 weeks, the animals were infected with hantavirus by inoculating a 1.2µL solution containing 100pfu hantavirus in each nasal cavity.
A group receiving 1µg CTB* (CTB (Cholera toxin subunit B) containing 0.1% CT (Cholera toxin)) served as the positive control for adjuvant. Groups receiving 3µg vaccine alone or no treatment served as controls.
3 days after the infection, nasal wash and serum were collected. IgA antibody in the nasal wash and IgG antibody in the serum were assayed by ELISA method. The virus titer of the nasal wash was determined by a plaque assay using MDCK cells.
Meanwhile, mice were nasally immunized in the same fashion and inoculated with a 20µL solution containing a lethal dose of hantavirus (40LD₅₀). The survival rate of the animals was monitored.

### 3. Results

The mucosal adjuvant activity of ceramified surf clam shell powder was evaluated in the following manner. 3µg vaccine and 10µg to 100µg ceramified surf clam shell powder were nasally inoculated 6 weeks previous. This was followed by additional two nasal inoculations of the same doses of the vaccine and the adjuvant given at a 2-week interval. The IgA antibody responses in the nasal mucosa and serum IgG responses were assayed.
To determine if the adjuvant activity of the ceramified surf clam shell powder varies in a dose-dependent manner, the ceramified surf clam shell powder was given at two different doses of 10 µg and 100 µg. It turned out that 10 µg of the ceramified surf clam shell powder induced the IgA antibody responses in the nasal mucosa. The amount of IgA antibody induced in the nasal mucosa was dependent on the dose of ceramified surf clam shell powder: The adjuvant activity increased as the dose of the ceramified surf clam shell powder was increased to 100 µg.

The animals were infected with PR8 virus 2 weeks, after the third immunization with the same immunization conditions, by injecting a 1.2µL solution containing 100pfu virus in each nasal cavity. The nasal wash of the non-vaccinated control group had a titer of 10³ pfu/mL or higher. In contrast, the viral growth was completely suppressed in the groups receiving three nasal vaccinations each accompanied by the ceramified surf clam shell powder as adjuvant. The viral growth was not suppressed in the group receiving three vaccinations each consisting of 3µg vaccine alone.
These results indicate that the ceramified powder of surf clam shells act as a potent adjuvant.
The results are summarized in Table 6.

**(Table 6)**

| Vaccine | | Anti-HA IgA (Nasal wash) Mean (µg/mL) | Anti-HA IgG (serum) Mean (µg/mL) | Virus titer in nasal wash (Mean) (PFU/mL:10ⁿ) |
|---|---|---|---|---|
| Vaccine (µg) | Ceramified powder of surf clam shells (µg) | | | |
| 3 | 100 | 0.5 | 27.1 | <1* |
| 3 | 10 | 0.15 | 10.0 | <1* |
| 1 | CTB* | 1.20 | 23.1 | <1* |
| 3 | - | 0 | <0.1 | 3.3 |
| - | - | 0 | 0 | 3.2 |

| | | | | |
|---|---|---|---|---|
| *: p < 0.05 | | | | |

With regard to the induced subclass of serum IgG antibodies, IgG₁ antibody is predominantly induced as compared to IgG₂ₐ antibody, indicating immune responses of Th2 dominance. These results are shown in Table 7.

**(Table 7)**

| Vaccine | | Antibody titer (2ⁿ) (Mean) | | Th1/Th2 ratio (IgG₁/IgG₂ₐ) |
|---|---|---|---|---|
| Vaccine (µg) | Ceramified powder of surf clam shells (µg) | IgG₁ | IgG₂ₐ | |
| 3 | 100 | 15.0 | 7.6 | 1.97 |
| 3 | 10 | 14.4 | 7.1 | 2.03 |
| 1 | CTB* | 17.0 | 13.8 | 1.23 |

### Example 5: Infection of suckling mice

Suckling mice were infected in an infection experiment.
Suckling mice were nasally administered with an antigen (hantavirus) and poly(I:C) as an adjuvant and the antibody titer was measured.
Specifically, a group of animals were inoculated with 1µg antigen and 10µg poly(I:C) twice at a 3-week interval. The nasal wash and the serum antibody responses of the mice were assayed after 1 week. The IgA antibody titer in 1 mL of the nasal wash was determined to be about 200 ng/mL and the serum IgG antibody titer was 3 to 4 µg/mL.
Another group of animals were inoculated with 1µg antigen and 3µg poly(I:C) twice at a 3-week interval. The nasal wash and the serum antibody responses of the mice were assayed after 1 week. The IgA antibody titer in 1 mL of the nasal wash was determined to be about 100 ng/mL and the serum IgG antibody titer was 2 to 3 µg/mL.

### INDUSTRIAL APPLICABILITY

As set forth, the nasal administration of an inactivated flavivirus vaccine with poly(I:C) or a ceramified powder of surf clam shells as an adjuvant induces secretory IgA and IgG antibodies specific for flaviviruses and provides protection against viruses and other pathogens.
Under the current situation where no effective vaccine adjuvants are available, the present invention is of significant medical importance as it can be applied to mucosal vaccines against other pathogens.

## Claims

1. An inducer for inducing secretory IgA and IgG antibodies, comprising:
an inactivated antigen of a flavivirus; and
poly(I:C) or a ceramified powder of surf clam shells as an adjuvant.

2. The inducer for inducing secretory IgA and IgG antibodies according to claim 1, to be administered to a mucosa of a respiratory tract.

3. The inducer for inducing secretory IgA and IgG antibodies according to claim 1 or 2, wherein the flavivirus is West Nile virus, hantavirus, dengue virus, Japanese encephalitis virus, yellow fever virus, tick-borne encephalitis virus or Hepatitis C virus.

4. A method for inducing virus-specific IgA and IgG antibodies, comprising administering an inactivated antigen of a virus, along with poly(I:C) or a ceramified powder of surf clam shells as an adjuvant.

5. The method for inducing secretory IgA and IgG antibodies according to claim 4, wherein the administering involves administering to a mucosa of a respiratory tract.

6. The method for inducing virus-specific IgA and IgG antibodies according to claim 4 or 5, wherein the virus is West Nile virus, hantavirus, dengue virus, Japanese encephalitis virus, yellow fever virus, tick-borne encephalitis virus or Hepatitis C virus.

7. An adjuvant for an inactivated antigen of a flavivirus, comprising poly(I:C) or a ceramified powder of surf clam shells.

8. The adjuvant according to claim 7, wherein the virus is West Nile virus, hantavirus, dengue virus, Japanese encephalitis virus, yellow fever virus, tick-borne encephalitis virus or Hepatitis C virus.
